# EUROPEAN PATENT APPLICATION

(11) **EP 4 159 742 A1**
(43) Date of publication of application: **05.04.2023**
(21) Application number: 21818873.8
(22) Date of filing: 01.06.2021
(51) Int. Cl.: C07K 4/06, C12N 1/20

(54) **LACTIC ACID BACTERIA GROWTH PROMOTER**

(30) Priority: 02.06.2020 JP 2020096288
(71) Applicant: Asahi Group Holdings, Ltd., Tokyo 130-8602 (JP)
(72) Inventor: UCHIDA, Naoto, Moriya-shi, Ibaraki 302-0106 (JP); KIRITA, Masanobu, Moriya-shi, Ibaraki 302-0106 (JP)
(74) Representative: Müller-Boré & Partner Patentanwälte PartG mbB
(86) International application number: PCT/JP2021/020796
(87) International publication number: WO 2021/246392

(57) **Abstract**

Provided is a lactic acid bacteria growth promoter which has a growth promotion effect on lactic acid bacteria. The lactic acid bacteria growth promoter includes at least one peptide selected from the group consisting of: a first peptide having a total number of residues of not more than 9 and comprising a hydrophobic amino acid residue at the C-terminus; and a second peptide having a total number of residues of not more than 9. The ratio of the total number of hydrophobic amino acid residues and proline residues to the total number of residues is not less than 2/3.

## Description

### TECHNICAL FIELD

The present invention relates to a lactic acid bacteria growth promoter.

### BACKGROUND ART

Lactic acid bacteria are known to show complex auxotrophies. In culture of a lactic acid bacterium, the yield of the lactic acid bacterium obtained may vary due to variation of the quality of medium raw materials and the like. In relation to this, factors that promote the growth of lactic acid bacteria and the like have been proposed. For example, WO 2017/034011 proposes a growth agent for intestinal bacteria, including a particular cyclodipeptide. WO 2011/027719 proposes a growth promoter for a lactic acid bacterium belonging to the genus *Lactobacillus,* including κ-caseinoglycomacropeptide as an effective component.

### SUMMARY OF THE INVENTION

### PROBLEMS TO BE SOLVED BY THE INVENTION

An object of the present invention is to provide a lactic acid bacteria growth promoter having a growth-promoting action for a lactic acid bacterium.

### MEANS FOR SOLVING THE PROBLEMS

The following are specific means for solving the above problem, and the present invention includes the following embodiments. A first embodiment is a lactic acid bacteria growth promoter including at least one peptide selected from the group consisting of: a first peptide having a total number of residues of not more than 9 and including a hydrophobic amino acid residue at the C-terminus; and a second peptide having a total number of residues of not more than 9, wherein the ratio of the total number of hydrophobic amino acid residues and proline residues to the total number of residues is not less than 2/3.

In one embodiment, each hydrophobic amino acid residue may be a residue of an amino acid selected from the group consisting of alanine (A), glycine (G), valine (V), isoleucine (I), leucine (L), phenylalanine (F), tyrosine (Y), tryptophan (W), methionine (M), and cysteine (C), or may be a residue of an amino acid selected from the group consisting of alanine (A), glycine (G), phenylalanine (F), and tyrosine (Y).

In one embodiment, in the first peptide, the total number of residues may be not more than 6, and each hydrophobic amino acid residue may be a residue of an amino acid selected from the group consisting of phenylalanine (F) and tyrosine (Y). In one embodiment, in the first peptide, the ratio of the total number of hydrophobic amino acid residues and proline residues to the total number of residues may be not less than 2/3.

In one embodiment, in the second peptide, the ratio of the total number of hydrophobic amino acid residues and proline residues to the total number of residues may be not less than 3/4. In one embodiment, the second peptide may have a total number of residues of not more than 4. In one embodiment, the second peptide may be composed of a hydrophobic amino acid residue(s) and a proline residue(s). In one embodiment, the second peptide may comprise a hydrophobic amino acid residue at the C-terminus.

### EFFECT OF THE INVENTION

According to the present invention, a lactic acid bacteria growth promoter having a growth-promoting action for a lactic acid bacterium can be provided.

### BRIEF DESCRIPTION OF THE DRAWINGS

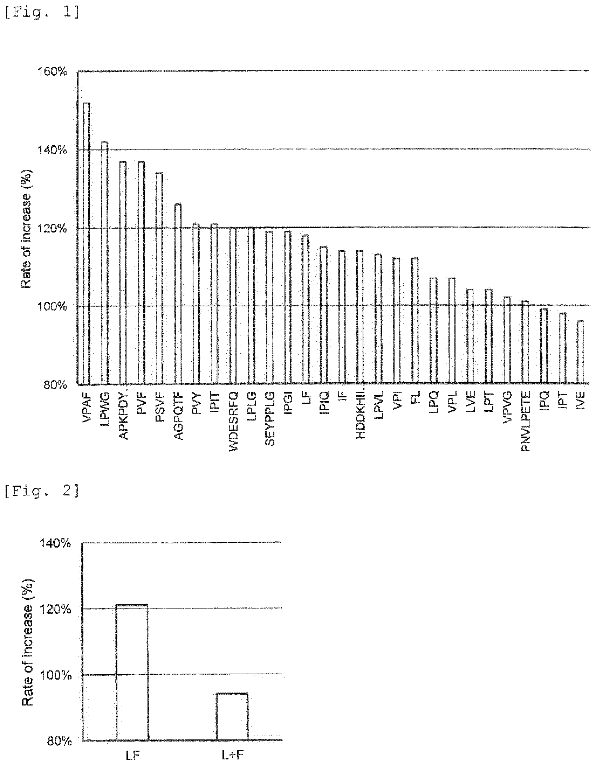

Fig. 1 is a diagram illustrating growth-promoting effects of various peptides on *Lactobacillus gasseri.*
Fig. 2 is a diagram illustrating growth-promoting effects of a dipeptide and an amino acid mixture on *Lactobacillus gasseri.*

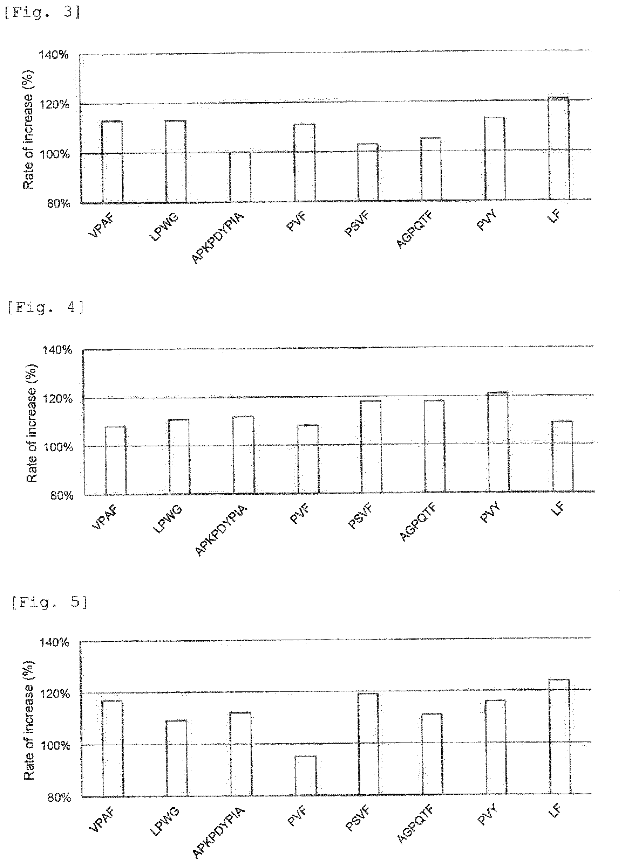

Fig. 3 is a diagram illustrating growth-promoting effects of various peptides on *Lactobacillus acidophilus.*
Fig. 4 is a diagram illustrating growth-promoting effects of various peptides on *Lactobacillus amylovorus.*
Fig. 5 is a diagram illustrating growth-promoting effects of various peptides on *Lactobacillus curvatus.*

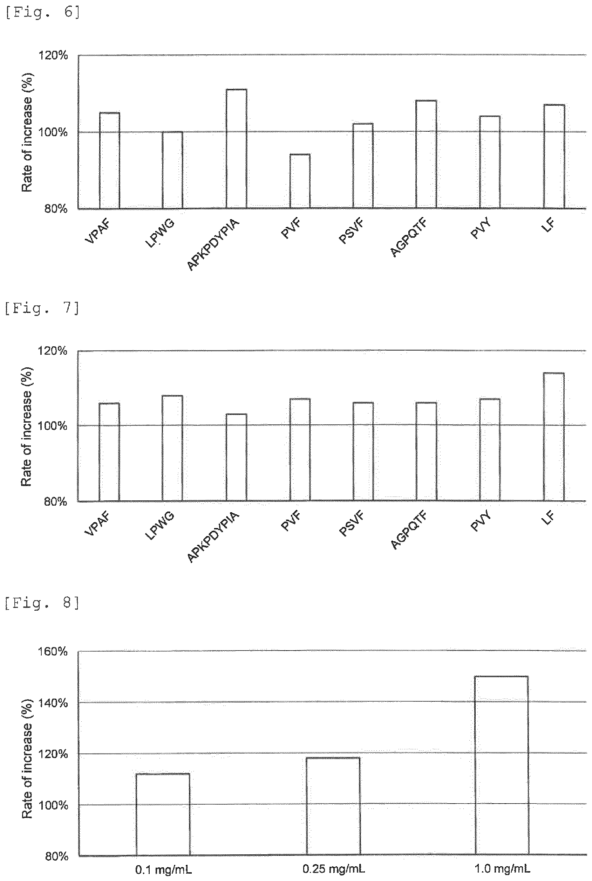

Fig. 6 is a diagram illustrating growth-promoting effects of various peptides on *Lactobacillus plantarum.*
Fig. 7 is a diagram illustrating growth-promoting effects of various peptides on *Lactobacillus brevis.*
Fig. 8 is a diagram illustrating a growth-promoting effect of a dipeptide on *Lactobacillus gasseri.*

### MODE FOR CARRYING OUT THE INVENTION

In the present description, unless otherwise specified, when a plurality of substances corresponding to a certain component are present in a composition, the content of the component in the composition means the total amount of the plurality of substances present in the composition. Embodiments of the present invention are described below in detail. The embodiments described below, however, are merely examples of lactic acid bacteria growth promoters for realization of the technological thought of the present invention. Therefore, the present invention is not limited to the lactic acid bacteria growth promoters described below.

### Lactic Acid Bacteria Growth Promoter

The lactic acid bacteria growth promoter may comprise at least one first peptide having a total number of residues of not more than 9 and comprising a hydrophobic amino acid residue at the C-terminus. The lactic acid bacteria growth promoter may comprise at least one second peptide having a total number of residues of not more than 9, wherein the ratio of the total number of hydrophobic amino acid residues and proline residues to the total number of residues in the peptide is not less than 2/3. Peptides having particular constitutions have a lactic acid bacteria growth action. By inclusion of at least one peptide selected from the group consisting of the first peptide and the second peptide having a lactic acid bacteria growth action, the lactic acid bacteria growth promoter can exhibit an excellent lactic acid bacteria growth-promoting effect.

Here, examples of the amino acids in the present description include the amino acids used for protein synthesis, and they are preferably L-amino acids. The "amino acids used for protein synthesis" means the 20 kinds of amino acids encoded as codons in the genes. The amino acids used for protein synthesis include hydrophobic amino acids, hydrophilic amino acids, and proline (P). The hydrophobic amino acids include aliphatic hydrophobic amino acids including alanine (A), glycine (G), valine (V), isoleucine (I), and leucine (L); aromatic hydrophobic amino acids including phenylalanine (F), tyrosine (Y), and tryptophan (W); and sulfur-containing hydrophobic amino acids including methionine (M) and cysteine (C). The hydrophilic amino acids include basic hydrophilic amino acids including lysine (K), arginine (R), and histidine (H); acidic hydrophilic amino acids including aspartic acid (D) and glutamic acid (E); and neutral hydrophilic amino acids including serine (S), threonine (T), asparagine (N), and glutamine (Q). Proline (P) is an imino acid, and may be referred to as a special amino acid in the present description.

The amino acids constituting a peptide contained in the lactic acid bacteria growth promoter may include the 20 kinds of amino acids used for protein synthesis; amino acids capable of constituting a protein, including not only the amino acids used for protein synthesis, but also amino acids generated by post-translational modification such as selenocysteine, pyrrolysine, N-formylmethionine, pyroglutamic acid, cystine, hydroxyproline, hydroxylysine, thyroxine, and O-phosphoserine; and amino acids that are normally not contained in a protein, such as β-alanine, sarcosine, ornithine, citrulline, creatine, γ-aminobutyric acid (GABA), opine, and trimethylglycine.

The total number of residues in each of the first peptide and the second peptide contained in the lactic acid bacteria growth promoter may be not more than 9 residues, or is preferably not more than 8 residues, not more than 7 residues, not more than 6 residues, not more than 5 residues, or not more than 4 residues. The total number of residues in each of the first peptide and the second peptide may be not less than 2 residues, or is preferably not less than 3 residues or not less than 4 residues. The first peptide and the second peptide may have the same total number of residues or different total numbers of residues.

The first peptide comprises a hydrophobic amino acid residue at the C-terminus. The hydrophobic amino acid residue present at the C-terminus of the first peptide may be at least one selected from the group consisting of alanine (A), glycine (G), valine (V), isoleucine (I), leucine (L), phenylalanine (F), tyrosine (Y), tryptophan (W), methionine (M), and cysteine (C). The hydrophobic amino acid residue present at the C-terminus of the first peptide is preferably at least one selected from the group consisting of alanine (A), glycine (G), phenylalanine (F), and tyrosine (Y), more preferably at least one selected from the group consisting of phenylalanine (F) and tyrosine (Y) .

In one embodiment, the first peptide may have a total number of residues of not more than 6, and may comprise at least one hydrophobic amino acid residue selected from the group consisting of phenylalanine (F) and tyrosine (Y) at the C-terminus. In the first peptide, the ratio of the total number of hydrophobic amino acid residues and proline residues to the total number of residues ((number of hydrophobic amino acid residues + number of proline residues) / total number of residues; which may be hereinafter referred to as "specific-residue ratio") may be not less than 2/3. The specific-residue ratio is preferably not less than 3/4.

The following are specific examples of the first peptide. However, the present invention is not limited by these. For example, the first peptide may include a peptide having the same amino acid sequence as an amino acid sequence in the following specific examples except that one or two arbitrary residues are inserted, deleted, substituted, and/or added, which peptide has a growth-promoting action for lactic acid bacteria. The lactic acid bacteria growth promoter may contain the first peptide as a single kind of peptide or as a combination of two or more kinds of peptides.

**[Table 1]**

| Peptide sequence | C-terminus | Total number of residues | Specific-residue ratio | SEQ ID NO: |
|---|---|---|---|---|
| VPAF | F | 4 | 4/4 | 1 |
| LPWG | G | 4 | 4/4 | 2 |
| APKPDYPIA | A | 9 | 7/9 | 3 |
| PVF | F | 3 | 3/3 | - |
| PSVF | F | 4 | 314 | 4 |
| AGPQTF | F | 6 | 416 | 5 |
| PVY | Y | 3 | 3/3 | - |
| LPLG | G | 4 | 4/4 | 7 |
| SEYPPLG | G | 7 | 5/7 | 8 |
| IPGI | I | 4 | 4/4 | 9 |
| LF | F | 2 | 2/2 | - |
| IF | F | 2 | 2/2 | - |
| LPVL | L | 4 | 4/4 | 11 |
| VPI | I | 3 | 3/3 | - |
| FL | L | 2 | 2/2 | - |
| VPL | L | 3 | 3/3 | - |
| VPVG | G | 4 | 4/4 | 12 |

In the second peptide, the total number of residues is not more than 9, and the ratio of the total number of hydrophobic amino acid residues and proline residues to the total number of residues in the peptide (specific-residue ratio) is not less than 2/3. The specific-residue ratio is preferably not less than 3/4, or 1. Here, the specific-residue ratio of 1 means that the second peptide is composed only of a hydrophobic amino acid residue(s) and a proline residue(s). The total number of residues in the second peptide may be not more than 6, or is preferably not more than 4. The total number of residues in the second peptide may be not less than 2, or is preferably not less than 3. The second peptide may include a hydrophobic amino acid residue at the C-terminus.

The hydrophobic amino acid residue(s) in the second peptide may be at least one selected from the group consisting of alanine (A), glycine (G), valine (V), isoleucine (I), leucine (L), phenylalanine (F), tyrosine (Y), tryptophan (W), methionine (M), and cysteine (C). The hydrophobic amino acid residue(s) in the second peptide is/are preferably at least one selected from the group consisting of alanine (A), glycine (G), phenylalanine (F), and tyrosine (Y), more preferably at least one selected from the group consisting of phenylalanine (F) and tyrosine (Y) .

The following are specific examples of the second peptide. However, the present invention is not limited by these. For example, the second peptide may include a peptide having the same amino acid sequence as an amino acid sequence in the following specific examples except that one or two arbitrary residues are inserted, deleted, substituted, and/or added, which peptide has a growth-promoting action for lactic acid bacteria. The lactic acid bacteria growth promoter may contain the second peptide as a single kind of peptide or as a combination of two or more kinds of peptides.

**[Table 2]**

| Peptide sequence | C-terminus | Total number of residues | Specific-residue ratio | SEQ ID NO: |
|---|---|---|---|---|
| VPAF | F | 4 | 4/4 | 1 |
| LPWG | G | 4 | 4/4 | 2 |
| APKPDYPIA | A | 9 | 7/9 | 3 |
| PVF | F | 3 | 3/3 | - |
| PSVF | F | 4 | 3/4 | 4 |
| AGPQTF | F | 6 | 4/6 | 5 |
| PVY | Y | 3 | 3/3 | - |
| IPIT | T | 4 | 3/4 | 6 |
| LPLG | G | 4 | 4/4 | 7 |
| SEYPPLG | G | 7 | 5/7 | 8 |
| IPGI | I | 4 | 4/4 | 9 |
| LF | F | 2 | 2/2 | - |
| IPIQ | Q | 4 | 3/4 | 10 |
| IF | F | 2 | 2/2 | - |
| LPVL | L | 4 | 4/4 | 11 |
| VPI | I | 3 | 3/3 | - |
| FL | L | 2 | 2/2 | - |
| VPL | L | 3 | 313 | - |
| LVE | E | 3 | 2/3 | - |
| LPT | T | 3 | 213 | - |
| VPVG | G | 4 | 4/4 | 12 |

The lactic acid bacteria growth promoter may further include at least one other peptide having a lactic acid bacteria growth action in addition to the at least one selected from the group consisting of the first peptide and the second peptide. The following are specific examples of the other peptide. However, the present invention is not limited by these. The lactic acid bacteria growth promoter may contain the other peptide as a single kind of peptide or as a combination of two or more kinds of peptides.

**[Table 3]**

| Peptide sequence | C-terminus | Total number of residues | Specific-residue ratio | SEQ ID NO: |
|---|---|---|---|---|
| WDESRFQ | Q | 7 | 2/7 | 13 |
| HDDKHIIVD | D | 9 | 3/9 | 14 |
| PNVLPETE | E | 8 | 4/8 | 15 |

Each peptide contained in the lactic acid bacteria growth promoter may be selected by, for example, difference analysis of components for a lactic acid bacteria medium among different production lots. The difference analysis of the components may be carried out using, for example, liquid chromatography - mass spectrometry (LC-MS).

Examples of the lactic acid bacterium in the present description include species belonging to the genus *Lactobacillus* or *Lactococcus,* such as *Lactobacillus gasseri* (for example, FERM BP-11331), *Lactobacillus acidophilus* (FERM BP-4981), *Lactobacillus amylovorus* (FERM BP-11255), *Lactobacillus curvatus* (NITE P-02033), *Lactobacillus plantarum, Lactobacillus brevis, Lactobacillus casei, Lactobacillus paracasei, Lactobacillus rhamnosus, Lactobacillus helveticus, Lactobacillus fermentum, Lactobacillus brevis, Lactobacillus plantarum, Lactobacillus salivarius, Lactobacillus reuteri, Lactobacillus johnsonii, Lactobacillus jensenii, Lactobacillus crispatus, Lactobacillus delbrueckii, Lactobacillus zeae, Lactobacillus gallinarum,* and *Lactococcus lactis.* The lactic acid bacterium may be preferably at least one selected from the group consisting of these. The lactic acid bacterium is more preferably at least one selected from the group consisting of *Lactobacillus gasseri, Lactobacillus acidophilus, Lactobacillus amylovorus, Lactobacillus curvatus, Lactobacillus plantarum, Lactobacillus brevis.*

The lactic acid bacteria growth-promoting effect by the lactic acid bacteria growth promoter may be, for example, more than 100%, or may be preferably not less than 105%, not less than 110%, or not less than 120% when the growth rate of a control is taken as 100%. The lactic acid bacteria growth-promoting effect can be evaluated by adding the lactic acid bacteria growth promoter to a medium at 0.25 mg/mL, carrying out anaerobic culture at 37°C for 18 hours, and then investigating the rate of increase in the number of bacterial cells.

Each peptide contained in the lactic acid bacteria growth promoter may be obtained by, for example, allowing an appropriate protease to act on a protein such as an edible protein. The peptide may also be obtained by separation from a meat extract, yeast extract, or the like. The peptide may also be obtained by chemical synthesis. For the chemical synthesis of the peptide, a method usually used may be appropriately employed.

The lactic acid bacteria growth promoter may be used for production of a lactic acid bacterium, production of a fermentation product using a lactic acid bacterium, or the like. In the production of a lactic acid bacterium, for example, the lactic acid bacterium is cultured in an appropriate medium supplemented with a predetermined amount of the lactic acid bacteria growth promoter. The amount of the lactic acid bacteria growth promoter added to the medium may be, for example, 0.001 mg/mL to 25 mg/mL, and is preferably 0.01 mg/mL to 10 mg/mL, more preferably 0.1 mg/mL to 2.5 mg/mL. When necessary, the medium may be supplemented with a predetermined amount(s) of a meat extract, a yeast extract, a soy milk, a non-fat dry milk, sucrose, an inorganic salt, and/or the like that is/are appropriately selected. Regarding the culture conditions, for example, the culture may be carried out at a temperature of about 25°C to about 40°C at a pH of about 5 to about 6.5 under an anaerobic condition in a nitrogen gas atmosphere or the like. The initial concentration of the lactic acid bacterium may be, for example, not less than 1×10⁴ cells/mL-medium, or is preferably not less than 1×10⁵ cells/mL-medium. The culture period may be, for example, not less than 4 hours, or is preferably 6 hours to 48 hours. The culture method may be appropriately selected from culture methods that are usually used such as static culture, spinner culture, and shake culture. The lactic acid bacterium can be recovered from the resulting culture using a separation apparatus such as a centrifuge.

Examples of the fermentation product using the lactic acid bacterium include fermented milks and silages. The fermented milks can be obtained by adding the lactic acid bacterium and the lactic acid bacteria growth promoter to a milk, and allowing lactic acid fermentation of the milk. A silage is an animal feed prepared by filling an early-harvested feed crop (grass, maize, or the like) into a silo, allowing lactic acid fermentation of the crop, and then storing the resulting product. By adding the lactic acid bacteria growth promoter to the feed crop during the production of the silage, the lactic acid bacterium may be efficiently grown to promote the lactic acid fermentation. By this, growth of putrefactive bacteria can be effectively suppressed to enable production of a high-quality silage.

Thus, the present invention includes use of a lactic acid bacteria growth promoter in the production of a lactic acid bacterium, or in the production of a fermented product using a lactic acid bacterium. As other modes, the present invention also includes use of at least one peptide selected from the group consisting of the first peptide and the second peptide in the production of a lactic acid bacteria growth promoter, use of at least one peptide selected from the group consisting of the first peptide and the second peptide in culturing of a lactic acid bacterium, and at least one peptide selected from the group consisting of the first peptide and the second peptide to be used for culturing of a lactic acid bacterium.

### EXAMPLES

The present invention is described below more concretely by way of Examples. However, the present invention is not limited by these Examples.

### (1) Providing of Medium for Evaluation

Nitrogen sources in MRS medium were reduced to find a medium composition that achieves a total bacterial count of about 1.0×10⁸ cell/mL to 1.0×10⁹ cell/mL after culturing of each of the six bacterial strains *Lactobacillus gasseri* (FERM BP-11331), *Lactobacillus acidophilus* (FERM BP-4981), *Lactobacillus amylovorus* (FERM BP-11255), *Lactobacillus curvatus* (NITE P-02033), *Lactobacillus plantarum,* and *Lactobacillus brevis.* This composition, named "modified MRS medium", was used for the evaluation. Here, for the composition of the MRS medium, a composition described in a medium catalog provided by BD (Becton, Dickinson and Company) was used as a reference. The compositions of the MRS medium and the modified MRS medium are as follows.

**[Table 4]**

| | Distribution source | MRS medium (g/L) | Modified MRS medium (g / L) |
|---|---|---|---|
| Proteose Peptone No.3 | BD Difco | 10.00 | 2.50 - 10.00 |
| Beef extract | BD Difco | 10.00 | - |
| Yeast extract | BD Difco | 5.00 | - |
| Glucose (Dextrose) | Wako | 20.00 | 20.00 |
| Tween 80 (Polysorbate 80) | Wako | 1.00 | 1.00 |
| Diammonium hydrogen citrate | Wako | 2.00 | 2.00 |
| Sodium acetate | Nippon Synthetic Chemical Industry | 5.00 | 5.00 |
| Dipotassium hydrogen phosphate | Taihei Chemical Industry | 2.00 | 2.00 |
| Magnesium sulfate | Tomita Pharmaceutical | 0.10 | 0.10 |
| Manganese sulfate | Wako | 0.05 | 0.05 |

### Example 1

### Growth-Promoting Effect on Lactobacillus gasseri (FERM BP-11331)

By chemical synthesis, 28 kinds of candidate peptides having the sequences shown below were provided. Each candidate peptide was added to the modified MRS medium at 0.25 mg/mL to provide a medium to be evaluated. The lactic acid bacterium *Lactobacillus gasseri* was inoculated at 1.0×10⁷ cells/mL to the provided medium to be evaluated, and culture was carried out at a temperature of 37°C for a culture period of 18 hours. The resulting culture was collected, and subjected to measurement of the particle concentration (particles/mL) as a characteristic value that can be used as an index of the bacterial cell growth, using a particle count analyzer (CDA-1000, Sysmex). The rate of increase (%) was calculated relative to a control value obtained using a medium to which no peptide was added, which was taken as 100%. The results are shown in the following table.

**[Table 5]**

| Peptide sequence | | Particle concentration (x10⁸ / mL) | 4 Control (x10⁸ / mL) | Rate of increase |
|---|---|---|---|---|
| VPAF | Val-Pro-Ala-Phe | 2.62 | 1.73 | 152% |
| LPWG | Leu-Pro-Trp-Gly | 2.45 | 1.73 | 142% |
| APKPDYPIA | Ala-Pro-Lys-Pro-Asp-Tyr-Pro-Ile-Ala | 2.37 | 1.73 | 137% |
| PVF | Pro-Val-Phe | 2.51 | 1.83 | 137% |
| PSVF | Pro-Ser-Val-Phe | 2.32 | 1.73 | 134% |
| AGPQTF | Ala-Gly-Pro-Gln-Thr-Phe | 2.50 | 1.98 | 126% |
| PVY | Pro-Val-Tyr | 227 | 1.88 | 121% |
| IPIT | Ile-Pro-Ile-Thr | 2.23 | 1.84 | 121% |
| WDESRFQ | Trp-As p-Glu-S er-Arg-P he-GI n | 236 | 1.98 | 120% |
| LPLG | Leu-Pro-Leu-Gly | 2.20 | 1.84 | 120% |
| SEYPPLG | Ser-Glu-Tyr-Pro-Pro-Leu-Gly | 2.36 | 1.98 | 119% |
| IPGI | Ile-Pro-Gly-Ile | 2 19 | 1.84 | 119% |
| LF | Leu-Phe | 2.49 | 2.12 | 118% |
| IPIQ | Ile-Pro-Ile-Gln | 2.11 | 1.84 | 115% |
| IF | Ile-Phe | 2.01 | 1.76 | 114% |
| HDDKHIIVD | His-Asp-Asp-Lys-His-Ile-Ile-Val-Asp | 2.25 | 1.98 | 114% |
| LPVL | Leu-Pro-Val-Leu | 2.07 | 1.84 | 113% |
| VPI | Val-Pre-Ile | 2.11 | 1.88 | 112% |
| FL | Phe-Leu | 1.98 | 1.76 | 112% |
| LPQ | Leu-Pro-Gin | 1.97 | 1.83 | 107% |
| VPL | Val-Pro-Leu | 2.01 | 1.88 | 107% |
| LVE | Leu-Val-Glu | 1.96 | 1.88 | 104% |
| LPT | Leu-Pro-Thr | 1.90 | 1.83 | 104% |
| VPVG | Val-Pro-Val-Gly | 1.76 | 1.73 | 102% |
| PNVLPETE | Pro-Asn-Val-Leu-Pro-Glu-Thr-Glu | 2,00 | 1.98 | 101% |
| IPQ | Ile-Pro-Gln | 1.82 | 1.83 | 99% |
| IPT | Ile-Pro-Thr | 1.73 | 1.83 | 98% |
| IVE | Ile-Val-Glu | 1.81 | 1.88 | 96% |

### Comparative Example 1

Using the dipeptide LF (Leu-Phe) and an equimolar mixture of leucine (L) and phenylalanine (F), culture was carried out in the same manner as in Example 1, and the growth-promoting effects of the dipeptide and the amino acid mixture on the lactic acid bacterium were evaluated.

The results are shown in the following table.

**[Table 6]**

| | Particle concentration (x10⁸ / mL) | Control (x10⁸ / mL) | Rate of increase |
|---|---|---|---|
| LF | 3.88 | 3.21 | 121% |
| L+F | 3.01 | 3.21 | 94% |

### Example 2

### Growth-Promoting Effect on Lactobacillus acidophilus (FERM BP-4981)

For eight kinds of candidate peptides having the sequences shown below, the growth-promoting effect on a lactic acid bacterium was evaluated in the same manner as in Example 1 except that *Lactobacillus acidophilus* was used instead of *Lactobacillus gasseri.* The results are shown in the following table.

**[Table 7]**

| Peptide sequence | Particle concentration (x10⁷ / mL) | Control (x10⁷ / mL) | Rate of increase |
|---|---|---|---|
| VPAF | 10.4 | 9.16 | 113% |
| LPWG | 10.3 | 9.16 | 113% |
| APKPDYPIA | 9.73 | 9.70 | 100% |
| PVF | 10.1 | 9.16 | 111% |
| PSVF | 10.0 | 9.70 | 103% |
| AGPQTF | 10.2 | 9.70 | 105% |
| PVY | 11.0 | 9.70 | 113% |
| LF | 8.75 | 7.25 | 121% |

### Example 3

### Growth-Promoting Effect on Lactobacillus amylovorus (FERM BP-11255)

For eight kinds of candidate peptides having the sequences shown below, the growth-promoting effect on a lactic acid bacterium was evaluated in the same manner as in Example 1 except that *Lactobacillus amylovorus* was used instead of *Lactobacillus gasseri.* The results are shown in the following table.

**[Table 8]**

| Peptide sequence | Particle concentration (x10⁸ / mL) | Control (x10⁸ / mL) | Rate of increase |
|---|---|---|---|
| VPAF | 2.07 | 1.92 | 108% |
| LPWG | 2.13 | 1.92 | 111% |
| APKPDYPIA | 1.83 | 1.63 | 112% |
| PVF | 2.07 | 1.92 | 108% |
| PSVF | 1.92 | 1.63 | 118% |
| AGPQTF | 1.93 | 1.63 | 118% |
| PVY | 1.98 | 1.63 | 121% |
| LF | 2.08 | 1.91 | 109% |

### Example 4

### Growth-Promoting Effect on Lactobacillus curvatus (NITE P-02033)

For eight kinds of candidate peptides having the sequences shown below, the growth-promoting effect on a lactic acid bacterium was evaluated in the same manner as in Example 1 except that *Lactobacillus curvatus* was used instead of *Lactobacillus gasseri.* The results are shown in the following table.

**[Table 9]**

| Peptide sequence | Particle concentration (x10⁸ / mL) | Control (x10⁸ / mL) | Rate of increase |
|---|---|---|---|
| VPAF | 2.15 | 1.82 | 117% |
| LPWG | 1.97 | 1.82 | 109% |
| APKPDYPIA | 1.60 | 1.42 | 112% |
| PVF | 1.71 | 1.82 | 95% |
| PSVF | 1.68 | 1.42 | 119% |
| AGPQTF | 1.58 | 1.42 | 111% |
| PVY | 1.64 | 1.42 | 116% |
| LF | 2.22 | 1.82 | 124% |

### Example 5

### Growth-Promoting Effect on Lactobacillus plantarum

For eight kinds of candidate peptides having the sequences shown below, the growth-promoting effect on a lactic acid bacterium was evaluated in the same manner as in Example 1 except that *Lactobacillus plantarum* was used instead of *Lactobacillus gasseri.* The results are shown in the following table.

**[Table 10]**

| Peptide sequence | Particle concentration (x10⁹ / mL) | Control (x10⁹ / mL) | Rate of increase |
|---|---|---|---|
| VPAF | 3.27 | 3.10 | 105% |
| LPWG | 3.09 | 3.10 | 100% |
| APKPDYPIA | 3.45 | 3.10 | 111% |
| PVF | 2.92 | 3.10 | 94% |
| PSVF | 3.17 | 3.10 | 102% |
| AGPQTF | 3.36 | 3.10 | 108% |
| PVY | 3.23 | 3.10 | 104% |
| LF | 3.32 | 3.10 | 107% |

### Example 6

### Growth-Promoting Effect on Lactobacillus brevis

For eight kinds of candidate peptides having the sequences shown below, the growth-promoting effect on a lactic acid bacterium was evaluated in the same manner as in Example 1 except that *Lactobacillus brevis* was used instead of *Lactobacillus gasseri.* The results are shown in the following table.

**[Table 11]**

| Peptide sequence | Particle concentration (x10⁹ / mL) | Control (x10⁹ / mL) | Rate of increase |
|---|---|---|---|
| VPAF | 1.16 | 1.09 | 106% |
| LPWG | 1.18 | 1.09 | 108% |
| APKPDYPIA | 1.12 | 1.09 | 103% |
| PVF | 1.17 | 1.09 | 107% |
| PSVF | 1.16 | 1.09 | 106% |
| AGPQTF | 1.16 | 1.09 | 106% |
| PVY | 1.17 | 1.09 | 107% |
| LF | 1.24 | 1.09 | 114% |

### Example 7

Using a dipeptide (LF) as the peptide, the growth-promoting effect on a lactic acid bacterium was evaluated in the same manner except that the concentration of the dipeptide added was 0.1 mg/mL, 0.25 mg/mL, or 1.0 mg/mL. The results are shown in the following table.

**[Table 12]**

| Addition concentration (mg/mL) | Particle concentration (x10⁸ / mL) | Control (x10⁸ / mL) | Rate of increase |
|---|---|---|---|
| 0.1 | 2.38 | 2.12 | 112% |
| 0.25 | 2.49 | 2.12 | 118% |
| 1.0 | 3.17 | 2.12 | 150% |

The disclosure of Japanese Patent Application No. 2020-096288 (filing date: June 2, 2020) is incorporated herein by reference in its entirety. All documents, patent applications, and technical standards cited in the present description are incorporated herein by reference to the same extent as in cases where the individual documents, patent applications, and technical standards are specifically and individually described to be incorporated by reference.

## Claims

1. A lactic acid bacteria growth promoter comprising at least one peptide selected from the group consisting of:
a first peptide having a total number of residues of not more than 9 and comprising a hydrophobic amino acid residue at the C-terminus; and
a second peptide having a total number of residues of not more than 9, wherein the ratio of the total number of hydrophobic amino acid residues and proline residues to the total number of residues is not less than 2/3.

2. The lactic acid bacteria growth promoter according to claim 1, wherein each hydrophobic amino acid residue is a residue of an amino acid selected from the group consisting of alanine (A), glycine (G), valine (V), isoleucine (I), leucine (L), phenylalanine (F), tyrosine (Y), tryptophan (W), methionine (M), and cysteine (C).

3. The lactic acid bacteria growth promoter according to claim 1, wherein each hydrophobic amino acid residue is a residue of an amino acid selected from the group consisting of alanine (A), glycine (G), phenylalanine (F), and tyrosine (Y).

4. The lactic acid bacteria growth promoter according to any one of claim 1 to claim 3, wherein the first peptide has a total number of residues of not more than 6, and each hydrophobic amino acid residue is a residue of an amino acid selected from the group consisting of phenylalanine (F) and tyrosine (Y).

5. The lactic acid bacteria growth promoter according to any one of claim 1 to claim 4, wherein in the first peptide, the ratio of the total number of hydrophobic amino acid residues and proline residues to the total number of residues is not less than 2/3.

6. The lactic acid bacteria growth promoter according to any one of claim 1 to claim 5, wherein in the second peptide, the ratio of the total number of hydrophobic amino acid residues and proline residues to the total number of residues is not less than 3/4.

7. The lactic acid bacteria growth promoter according to any one of claim 1 to claim 6, wherein the second peptide has a total number of residues of not more than 4.

8. The lactic acid bacteria growth promoter according to any one of claim 1 to claim 7, wherein in the second peptide is composed of a hydrophobic amino acid residue(s) and a proline residue(s).

9. The lactic acid bacteria growth promoter according to any one of claim 1 to claim 8, wherein the second peptide comprises a hydrophobic amino acid residue at the C-terminus.
